# EUROPEAN PATENT APPLICATION

(11) **EP 4 631 610 A1**
(43) Date of publication of application: **15.10.2025**
(21) Application number: 22967919.6
(22) Date of filing: 05.12.2022
(51) Int. Cl.: B01F 23/43, B01F 23/41, B01F 27/2122, B01F 27/91, A61K 9/16, A61K 9/00, A61K 38/09, B01F 101/22

(54) **EMULSION HOMOGENIZATION APPARATUS AND METHOD FOR MANUFACTURING SUSTAINED-RELEASE MICROSPHERES HAVING IMPROVED DRUG ENCAPSULATION RATE**

(71) Applicant: Daewoong Pharmaceutical Co., Ltd., Hwaseong-si, Gyenoggi-do 18623 (KR)
(72) Inventor: LEE, Tae Ho, Yongin-si, Gyeonggi-do 16846 (KR); BAIK, Hye Jung, Seoul 06787 (KR); KIM, Jin Ho, Seoul 07981 (KR); BAE, Byoung Chan, Yongin-si, Gyeonggi-do 16843 (KR); KIM, Jun Sik, Yongin-si, Gyeonggi-do 16853 (KR); KIM, Gwan Young, Yongin-si, Gyeonggi-do 16824 (KR)
(74) Representative: Fuchs Patentanwälte Partnerschaft mbB
(86) International application number: PCT/KR2022/019604
(87) International publication number: WO 2024/122661

(57) **Abstract**

An emulsion homogenization apparatus, according to an embodiment of the present invention, comprises: a stirring container; a stirring unit provided with a stirring shaft and stirring blades and installed in the stirring container so as to be able to rotate; and a medicinal fluid injection part having a discharge port through which a main ingredient aqueous solution containing a drug is discharged in the form of droplets, the discharge port being installed in the stirring container, between the bottom surface of the stirring container and the stirring blades, so as to face the stirring blades.

## Description

### [Technical Field]

The present invention relates to an emulsion homogenization apparatus and a method of manufacturing sustained-release microspheres having an improved drug encapsulation rate, and more particularly, to an emulsion homogenization apparatus capable of increasing the encapsulation rate of a drug into microspheres through homogenization of a W1/O emulsion, and a method of manufacturing sustained-release microspheres having an improved drug encapsulation rate.

### [Background Art]

A sustained-release injection formulation refers to an injection formulation that is formulated so that a drug may be released continuously and uniformly while maintaining biological activity in the body when injected subcutaneously or intramuscularly.

Conventional preparation methods for sustained-release injection formulations include a coacervation method, a melt injection method, a spray drying method, and a solvent evaporation method. Among these methods, solvent evaporation methods, which are classified into a double emulsion evaporation method (W/O/W emulsion) and a single emulsion evaporation method (O/W emulsion), are the most commonly used.

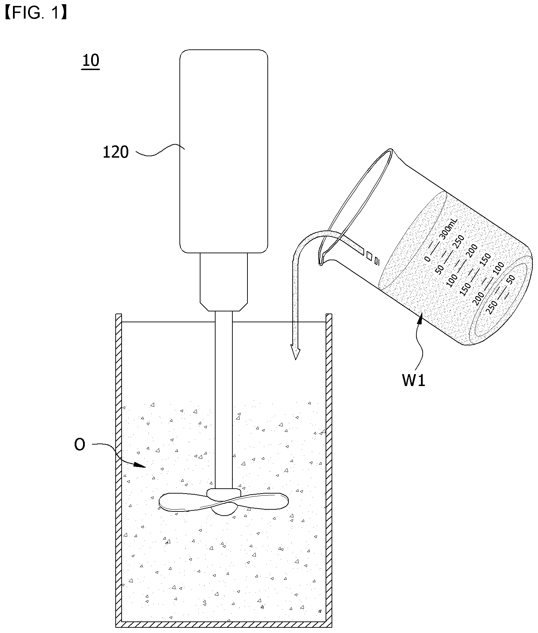

FIG. 1 shows a schematic diagram illustrating a stirring apparatus according to the related art.

Referring to FIG. 1, in the conventional microsphere manufacturing process, when forming a W1/O emulsion, a main ingredient aqueous solution W1 containing a drug and a polymer oil solution O are stirred and homogenized in a stirring container 10 by a stirring apparatus 20. Here, the main ingredient aqueous solution W1 is a water phase, and the polymer oil solution O is an oil phase.

Conventionally, after the polymer oil solution O is placed in the stirring container 10, the main ingredient aqueous solution W1 is added to the stirring container 10.

However, due to the difference in specific gravity between methylene chloride (MC), which is a solvent of the polymer oil solution O, and water, which is a solvent of the main ingredient aqueous solution, layer separation occurs, and the main ingredient aqueous solution W1 is located on the upper surface of the polymer oil solution O.

Thereafter, the stirring apparatus 20 rotates to stir the main ingredient aqueous solution W1 and the polymer oil solution O, thereby homogenizing the mixture of the main ingredient aqueous solution W1 and the polymer oil solution O.

During the homogenization using the stirring apparatus 20, when the volume of the mixture of the polymer oil phase solution O (oil phase) and the main ingredient aqueous solution W1 (water phase) is small, encapsulation efficiency does not significantly depend on the mixing method. However, as the volume of the mixture increases, the mixing homogeneity of the main ingredient aqueous solution W1 decreases due to the high viscosity of the polymer oil phase solution O.

In order for the high-viscosity polymer oil solution O and the main ingredient aqueous solution W1 to be mixed homogeneously, the rotation speed (rpm) and stirring time of the stirring apparatus 20 must be increased. In this case, due to heat generation, the solvent of the polymer oil solution O, methylene chloride (MC) (boiling point: 39 °C to 40 °C), boils and volatilizes, and as a result, the concentration of the polymer oil solution O changes.

In summary, when the volume of the mixture increases, the mixture is not stirred homogeneously, and when the mixture is not stirred homogeneously, emulsification of the mixture becomes difficult, and as a result, drug encapsulation efficiency decreases.

### [Disclosure]

### [Technical Problem]

An object of the present invention is to provide an emulsion homogenization apparatus in which a main ingredient aqueous solution W1 is injected in the form of droplets under stirring blades while an oily polymer aqueous solution O is being stirred in advance in a stirring container.

In addition, another object of the present invention is to provide an emulsion homogenization apparatus capable of homogenizing a W1/O emulsion by stirring a main ingredient aqueous solution W1 injected in the form of droplets with a polymer oil solution O, while being dispersed by the vortex of the polymer oil solution O.

In addition, still another object of the present invention is to provide an emulsion homogenization apparatus capable of increasing the encapsulation rate of a drug into microspheres through homogenization of a W1/O emulsion and a method of manufacturing sustained-release microspheres having an improved drug encapsulation rate.

### [Technical Solution]

To achieve the above-described objects, an emulsion homogenization apparatus according to one embodiment of the present invention includes: a stirring container; a stirring unit including a stirring shaft and stirring blades mounted on the stirring shaft, the stirring blades being rotatably provided within the stirring container; and a medicinal fluid injection part having a discharge port through which a main ingredient aqueous solution including a drug is discharged in the form of droplets.

**In** addition, the discharge port is located between a bottom surface of the stirring container and the stirring blades and disposed to face the stirring blades.

The medicinal fluid injection part may be provided to discharge the main ingredient aqueous solution in the form of droplets toward the stirring blades.

In addition, the medicinal fluid injection part may have a discharge port spaced apart from the stirring blades.

In addition, the emulsion homogenization apparatus may further include a control unit controlling the stirring unit and a medicinal fluid supply part.

In addition, the control unit may be provided to perform stirring of a polymer oil phase solution by rotating the stirring blades at a first speed, when the polymer oil phase solution is accommodated in the stirring container before the main ingredient aqueous solution is supplied through the medicinal fluid injection part.

In addition, the control unit may be provided to supply the main ingredient aqueous solution into the stirring container through the medicinal fluid supply part and increase the rotation speed of the stirring blades to a second speed greater than the first speed, while stirring the polymer oil phase solution.

In addition, the medicinal fluid injection part may include an injection tube.

In addition, the injection tube may be disposed in a state of being bent at least twice within the stirring container.

In addition, the bottom surface of the stirring container may be provided with a body through-hole at a position facing the stirring blades, and the medicinal fluid injection part may be inserted into the stirring container through the body through-hole, and the discharge port may be installed facing the stirring blades.

In addition, the stirring unit may have a stirring through-hole passing through the stirring shaft and the stirring blades, the medicinal fluid injection part may be disposed within the stirring unit along the stirring through-hole, and the discharge port may be provided to pass through the stirring through-hole and be exposed under the stirring blades.

In addition, a method of manufacturing sustained-release microspheres having an improved drug encapsulation rate according to one embodiment of the present invention includes: S1) a step of stirring a polymer oil phase solution O, which is an oil phase, using the emulsion homogenization apparatus of claim 1; S2) a step of injecting a main ingredient aqueous solution W1 in the form of droplets into the polymer oil phase solution O being stirred; S3) a step of forming a W1/O emulsion by a first homogenization process of the main ingredient aqueous solution W1 and the polymer oil phase solution O; S4) a step of injecting the W1/O emulsion in the form of droplets into a polymer aqueous solution W2 while stirring the polymer aqueous solution W2; S5) a step of forming a W1/O/W2 emulsion by a second homogenization process of the polymer aqueous solution W2 and the W1/O emulsion; and S6) a step of forming microspheres by an underwater drying process of the W1/O/W2 emulsion.

In Step S2, the droplets of the main ingredient aqueous solution W1 may be injected under the stirring unit of the emulsion homogenization apparatus and mixed with the polymer oil phase solution O, while being dispersed by the vortex of the polymer oil phase solution O.

In Step S2, the polymer oil phase solution O may be stirred at a speed 1.5 times faster than that of Step S1.

In Step S4, the W1/O emulsion may be injected for a preset time at a rate within a range that is 1/100 slower than the injection rate of the polymer aqueous solution W2 while the polymer aqueous solution W2 is flowing into the stirring container.

The polymer oil phase solution O may a solution in which a polymer material, polylactic acid (PLA) or poly lactic-co-glycolic acid (PLGA), is dissolved in methylene chloride (MC).

In addition, the polymer aqueous solution W2 may be a solution in which a polymer substance, polyvinyl alcohol (PVA), is dissolved in water for injection (WFI).

In addition, the main ingredient aqueous solution W1 may be a solution in which a drug is dissolved in WFI.

Herein, the drug may be leuprolide acetate, and the chemical formula of the leuprolide acetate may be (2S)-N-[(2S)-1-[[(2S)-1-[[(2S)-1-[[(2S)-1-[[(2R)-1-[[(2S)-1-[[(2*S*)-5-(diaminomethylideneamino)-1-[(2*S*)-2-(ethylcarbamoyl)pyrrolidin-1-yl]-1-oxopentan-2-yl]amino]-4-methyl-1-oxopentan-2-yl]amino]-4-methyl-1-oxopentan-2-yl]amino]-3-(4-hydroxyphenyl)-1-oxopropan-2-yl]amino]-3-hydroxy-1-oxopropan-2-yl]amino]-3-(1*H*-indol-3-yl)-1-oxopropan-2-yl]amino]-3-(1*H*-imidazol-5-yl)-1-oxopropan-2-yl]-5-oxopyrrolidine-2-carboxamide;[(8R,9S,10R,13S,14S,17R)-17-ethynyl-13-methyl-3-oxo-1,2,6,7,8,9,10,11,12,14,15,16-dodecahydrocyclopenta[a]phenanthren-17-yl] acetate.

### [Advantageous Effects]

As described above, the emulsion homogenization apparatus related to at least one embodiment of the present invention has the effects described below.

While a polymer oil phase solution O is being stirred in advance, a main ingredient aqueous solution can be injected in the form of droplets under stirring blades in a stirring container. At this time, the injected main ingredient aqueous solution droplets can be dispersed by the vortex of the polymer oil phase solution O being stirred and stirred with the polymer oil phase solution O. As a result, the main ingredient aqueous solution W1 and the polymer oil phase solution O can be homogeneously stirred, so that the W1/O emulsion can be homogenized.

In addition, a method of manufacturing sustained-release microspheres having an improved drug encapsulation rate using the emulsion homogenization apparatus can improve drug encapsulation rate by approximately twice compared to the conventional method through homogenization of the W1/O emulsion.

### [Description of Drawings]

FIG. 1 shows a schematic diagram illustrating a stirring apparatus according to the related art.
FIG. 2 shows a diagram for illustrating an operation state diagram of an emulsion homogenization apparatus according to one embodiment of the present invention.
FIGS. 3 to 5 are schematic diagrams illustrating the structure of emulsion homogenization apparatuses according to various embodiments.
FIG. 6 shows a flowchart for illustrating a method of manufacturing sustained-release microspheres having an improved drug encapsulation rate according to one embodiment of the present invention.

### [Modes of the Invention]

Hereinafter, with reference to the attached drawings, an emulsion homogenization apparatus and a method of manufacturing sustained-release microspheres having an improved drug encapsulation rate according to a preferred embodiment of the present invention will be described.

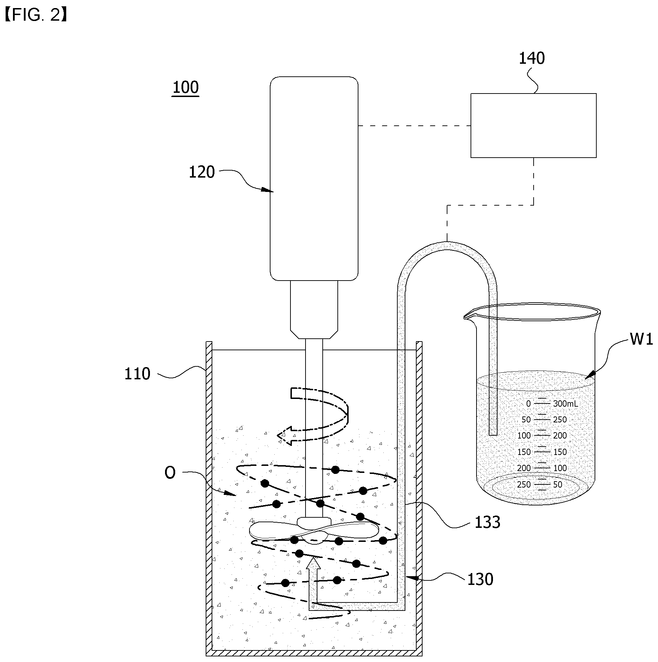

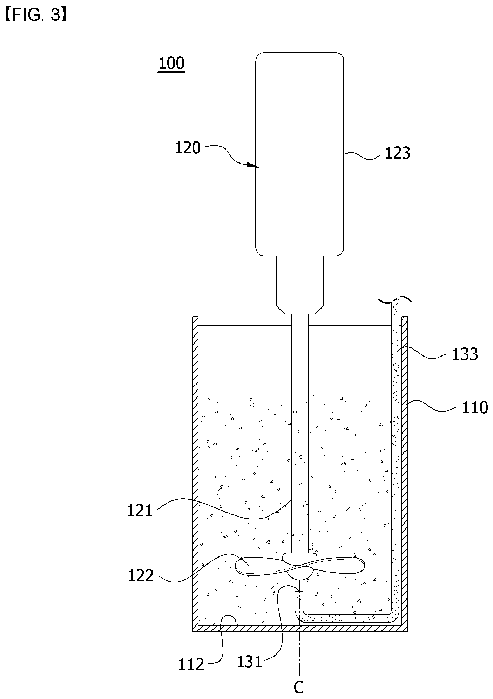

FIG. 2 shows a diagram for illustrating an operation state diagram of an emulsion homogenization apparatus according to one embodiment of the present invention, and FIGS. 3 to 5 are schematic diagrams illustrating the structure of emulsion homogenization apparatuses according to various embodiments.

Referring to FIGS. 2 and 3, an emulsion homogenization apparatus 100 according to one embodiment of the present invention includes a stirring container 110, a stirring unit 120, and a medicinal fluid injection part 130.

The stirring unit 120 includes a stirring shaft 121 and stirring blades 122 mounted on the stirring shaft 121. In addition, the stirring unit 120 is provided such that the stirring blades 122 may rotate within the stirring container 110. The stirring unit 120 may include a driving unit 123 (e.g., a motor, etc.) for rotating the stirring shaft 121.

As described through FIG. 1, the stirring container 110 and the stirring unit 120 are known components, and their detailed description will be omitted in the present embodiment.

The medicinal fluid injection part 130 is provided to supply a main ingredient aqueous solution W1 into the stirring container 110. Here, the main ingredient aqueous solution W1 is a solution in which a drug is dissolved in water for injection (WFI), and various types of drugs may be applied depending on the prepared type of injection.

The medicinal fluid injection part 130 has a discharge port 131 through which the main ingredient aqueous solution containing the drug is discharged in the form of droplets. In addition, the discharge port 131 is positioned between a bottom surface 112 of the stirring container 110 and the stirring blades 120, and the discharge port 131 is disposed facing the stirring blades 120.

The medicinal fluid injection part 130 may be disposed to discharge the main ingredient aqueous solution in the form of droplets toward the stirring blades 120. In addition, the medicinal fluid injection part 130 may be disposed such that the discharge port 131 is spaced apart in a predetermined distance from the stirring blades 120.

In addition, the medicinal fluid injection part 130 may include an injection tube 133. In the present embodiment, the medicinal fluid injection part 130 may include an injection tube 133 having a diameter in single-digit mm units (e.g., 3 mm to 5 mm). The discharge port 131 of the medicinal fluid injection part 130 may discharge the main ingredient aqueous solution in the form of droplets, and the medicinal fluid injection part 130 may be installed in the stirring container 110 such that the discharge port 131 faces the stirring blades 122 between the bottom surface 112 of the stirring container 110 and the stirring blades 122. For example, when the organic solvent constituting the polymer oil phase solution O is methylene chloride (MC), which has very low solubility in water, and when the main ingredient aqueous solution W1 is allowed to flow out from inside the polymer oil phase solution O through the injection tube 133, since the aqueous solution W1 cannot be mixed with the oil phase solution O due to a difference in polarity between the solutions, the main ingredient aqueous solution may be discharged in the form of droplets.

For example, referring to FIG. 3, the medicinal fluid injection part 130 may include an injection tube 133, and the injection tube 133 may be disposed in a state of being bent at least twice within the stirring container 110. For example, the injection tube 133 may be disposed in a hook shape within the stirring container 110.

At this time, the medicinal fluid injection part 130 is provided such that the discharge port 131 is positioned toward the top (in the direction toward the stirring unit) on the bottom surface 112 of the stirring container 110, specifically, under the stirring blades 122, and the discharge port 131 is disposed to be spaced apart from the stirring blades 122.

The discharge direction of the medicinal fluid injection part 130 is arranged to face upward from the bottom surface 112 of the stirring container 110. The discharge direction of the medicinal fluid injection part 130 refers to the direction in which the pressure by which the medicinal liquid is discharged faces. The discharge port 131 may be disposed so that its center is located within the rotation radius of the stirring blades 122, and may be disposed, for example, to be coaxial C with the stirring shaft 121 (see FIG. 3).

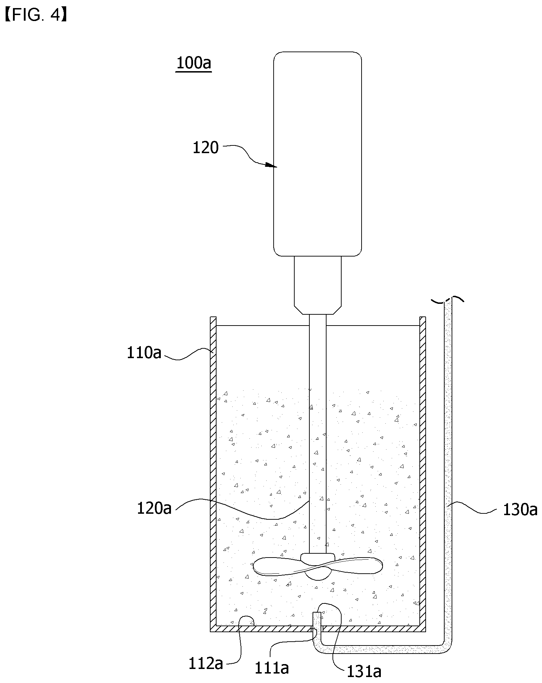

As another example, referring to FIG. 4, in an emulsion homogenization apparatus 100a, a medicinal fluid injection part 130a may be inserted into a stirring container 110a by passing through a main body through-hole 111a provided in a bottom surface 112a of the stirring container 110a so that a discharge port 131a is provided at a position spaced from the bottom of the stirring blades and facing the stirring blades.

In other words, the main body through-hole 111a may be provided in the bottom surface 112a of the stirring container 110a at a position facing the stirring blades, and the medicinal fluid injection part 130a may be inserted into the stirring container 110a by passing through the main body through-hole 111a, and the discharge port 131a may be installed to face the stirring blades.

In addition, the main body through-hole 111a may be provided in a size that allows the medicinal fluid injection part 130a to pass through. After the medicinal fluid injection part 130 is installed in the stirring container 110, the gap between the medicinal fluid injection part 130a and the main body through-hole 111a may be sealed to prevent leakage of the contents accommodated in the stirring container 110.

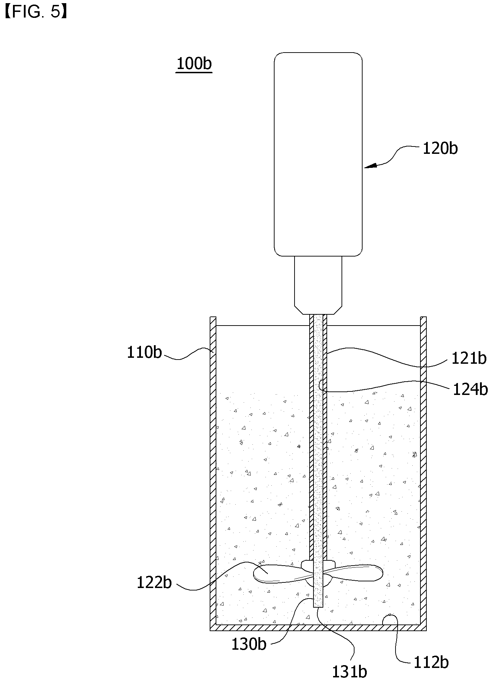

As another example, referring to FIG. 5, in an emulsion homogenization apparatus 100b, a stirring unit 120b may have a stirring through-hole 124b passing through a stirring shaft 121 and stirring blades 122b.

In addition, a medicinal fluid injection part 130b may be disposed within the stirring unit 120b along the stirring through-hole 124b, and a discharge port 131b may be provided to pass through the stirring through-hole 124b and be exposed under the stirring blades 122b. In other words, the medicinal fluid injection part 130b may be provided as an integral part with the stirring unit 120b. In the present embodiment, the stirring unit 120b may include a stirring through-hole 124b passing through the stirring shaft 121b and the stirring blades 122b. The medicinal fluid injection part 130b may be installed in the stirring unit 120b along the stirring through-hole 124b so that the discharge port 131b is exposed under the stirring blades 122b.

In addition, referring to FIG. 2, the emulsion homogenization apparatus 100 may further include a control unit 140 controlling the stirring unit 120 and the medicinal fluid injection part 130.

In addition, the control unit 140 may be provided to perform stirring of the polymer oil phase solution O by rotating the stirring blades 122 at a first speed when the polymer oil phase solution O is accommodated in the stirring container 110 before the main ingredient aqueous solution is supplied through the medicinal fluid injection part 130.

In addition, the control unit 140 may be provided to supply the main ingredient aqueous solution W1 into the stirring container 110 through the medicinal fluid injection part 130 and increase the rotation speed of the stirring blades 122 to a second speed greater than the first speed while stirring the polymer oil phase solution O. For example, the second speed may be 1.5 times faster than the first speed.

In the emulsion homogenization apparatuses 100, 100a, and 100b having the above-described structure, while the polymer oil phase solution O, which is an oil phase, is being stirred in advance in a stirring container 110, 110a, and 110b, the main ingredient aqueous solution W1 may be injected in the form of droplets under the stirring blades in the stirring container. At this time, the main ingredient aqueous solution W1 is dispersed by the vortex of the polymer oil phase solution O caused by the rotation of the stirring unit and mixed with the polymer oil phase solution O, and as a result, a W1/O emulsion may be homogenized.

Hereinafter, the effects of the present invention will be described through experimental examples of a method of manufacturing sustained-release microspheres in which a W1/O emulsion is manufactured using a conventional stirring apparatus 10 and the emulsion homogenization apparatus 100 of the present invention.

**[Table 1]**

| | Drug encapsulation rate (%) | Mean particle diameter (um) |
|---|---|---|
| Experiment 1 | 42.8 | 35.1 |
| Experiment 2 | 91.7 | 20.3 |
| Experiment 3 | 85.9 | 17.8 |
| Experiment 4 | 98.0 | 20.9 |
| Experiment 5 | 95.5 | 20.4 |

Table 1 compares the drug encapsulation rate and the mean particle diameter of the microparticles in the case of manufacturing the W1/O emulsion using the conventional stirring apparatus 10 (Experiment 1) and in the case of manufacturing the W1/O emulsion using the emulsion homogenization apparatus 100 of the present invention (Experiments 2 to 5). Experiments 1 to 5 were performed under the same conditions.

Experiment 1 was an experiment in which the main ingredient aqueous solution W1 was injected into the polymer oil phase solution O (see FIG. 1) to homogenize the polymer oil phase solution O and the main ingredient aqueous solution W1. As a result of Experiment 1, the mean particle diameter of the microparticles was 35.1 µm, and the drug encapsulation efficiency was 42.8%.

Experiments 2 to 5 in Table 2 were experiments using the emulsion homogenization apparatus 100 according to one embodiment of the present invention. Experiments 2 to 5 were experiments in which the main ingredient aqueous solution W1 in the form of droplets was injected under the polymer oil phase solution O that was being stirred through the medicinal fluid injection part 130 to homogenize the polymer oil phase solution O and the main ingredient aqueous solution W1.

As a result of Experiment 2, the mean particle diameter of the microspheres was 20.3 µm, and the drug encapsulation efficiency was 91.7%. As a result of Experiment 3, the mean particle diameter of the microspheres is 17.8 µm, and the drug encapsulation efficiency was 85.9%. As a result of Experiment 4, the mean particle diameter of the microspheres was 20.9 µm, and the drug encapsulation efficiency was 98.0%. As a result of Experiment 5, the mean particle diameter of the microspheres was 20.4 µm, and the drug encapsulation efficiency was 95.5%.

As a result of Experiments 2 to 5, the mean particle diameter of the microparticles ranged from 17.8 µm to 20.9 µm, and the drug encapsulation rate ranged from 85.9% to 98%. Compared to the results of Experiment 1, it can be seen that the drug encapsulation rate (average 92.78%) in the cases where the medicinal fluid injection part 130 was used (Experiments 2 to 5) increased by 2.16 times compared to the drug encapsulation rate (42.8%) in the case where the medicinal fluid injection part 130 was not used (Experiment 1), and the mean particle diameter decreased to approximately half the size.

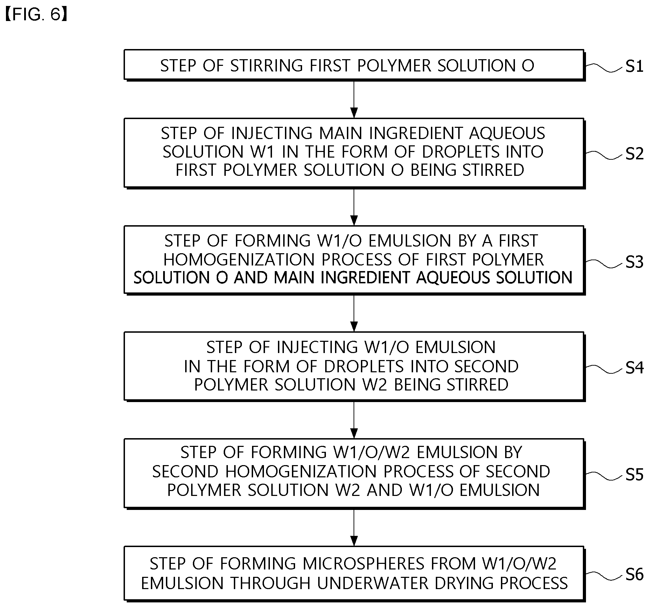

FIG. 6 shows a flowchart for illustrating a method of manufacturing sustained-release microspheres having an improved drug encapsulation rate according to one embodiment of the present invention.

Hereinafter, with reference to FIG. 6, the method of manufacturing sustained-release microspheres having an improved drug encapsulation rate according to a preferred embodiment of the present invention will be described.

In comparison with the conventional method in which a polymer oil phase solution O and a main ingredient aqueous solution W1 are mixed and the resulting mixed liquid is stirred to manufacture a W1/O emulsion, in the method of manufacturing sustained-release microspheres having an improved drug encapsulation rate according to one embodiment of the present invention, a polymer oil phase solution O is first stirred, and then the main ingredient aqueous solution W1 is injected into the polymer oil phase solution O being stirred.

The main ingredient aqueous solution W1 is discharged in the form of droplets from the bottom of the stirring container 110 toward the stirring blades 122 by the medicinal fluid injection part 130. The droplets of the main ingredient aqueous solution W1 are dispersed along the vortex of the polymer oil phase solution O by the rotational force of the stirring blades 122 and are mixed with the polymer oil phase solution O. Through the first homogenization process, the resulting mixed liquid of the main ingredient aqueous solution W1 and the polymer oil phase solution O is homogenized while the mixed liquid is being stirred by the stirring unit 120, thereby forming a W1/O emulsion.

The process of manufacturing a W1/O emulsion is described as follows with an experimental example.

First, the components and amounts of the polymer oil phase solution O and the main ingredient aqueous solution W1 used to manufacture a W1/O emulsion will be described.

**[Table 2]**

| Solution | Injection amount (g) |
|---|---|
| Polymer oil phase solution O | 1397.28 g |
| Main ingredient aqueous solution W1 | 158.72 g |

Table 2 shows the amounts of the polymer oil phase solution O and the main ingredient aqueous solution W1 used to prepare a W1/O emulsion in the first homogenization process.

When 1397.28 g of polymer oil phase solution O is injected, 158.72 g of main ingredient aqueous solution W1 is injected. The polymer oil phase solution O is injected in Step S1, and the main ingredient aqueous solution W1 is injected in the form of droplets from the bottom surface of the stirring container 110 toward the stirring blades 122 in Step S2.

The polymer oil phase solution O is a solution in which a polymer material, polylactic acid (PLA) or poly lactic-co-glycolic acid (PLGA), is dissolved in methylene chloride (MC).

In addition, the main ingredient aqueous solution W1 is a solution in which a drug is dissolved in WFI. The drug dissolved in WFI may vary depending on the type of injection to be prepared.

For example, when manufacturing a leuprolide depot injection that is used to treat endometriosis, uterine fibroids, prostate cancer, premenopausal breast cancer, and central precocious puberty, the drug included in the main ingredient aqueous solution W1 may be leuprolide acetate.

The chemical formula of the leuprolide acetate is (2S)-N-[(2S)-1-[[(2S)-1-[[(2S)-1-[[(2S)-1-[[(2R)-1-[[(2S)-1-[[(2S)-5-(diaminomethylideneamino)-1-[(2S)-2-(ethylcarbamoyl)pyrrolidin-1-yl]-1-oxopentan-2-yl]amino]-4-methyl-1-oxopentan-2-yl]amino]-4-methyl-1-oxopentan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide;[(8R,9S,10R,13S,14S,17R)-17-ethynyl-13-methyl-3-oxo-1,2,6,7,8,9,10,11,12,14,15,16-dodecahydrocyclopenta[a]phenanthren-17-yl] acetate.

**[Table 3]**

| W1/O emulsion manufacturing step | Stirring speed | Stirring time |
|---|---|---|
| S1- Stirring the polymer oil phase solution O in advance | 4000 rpm | 1 minute |
| S2- Stirring the main ingredient aqueous solution W1 while injecting the same | 6000 rpm | 3 minutes |
| S3- Further stirring after completion of Step S2 | 6000 rpm | 2 minutes |

Table 3 shows the stirring speed and stirring time at each step in the manufacture of W1/O emulsion.

Referring to Table 3 and FIG. 6, in Step S1, the stirring unit 120 rotates at 4000 rpm for one minute to stir the polymer oil phase solution O. Thereafter, the main ingredient aqueous solution W1 is injected in the form of droplets into the polymer oil phase solution O being stirred (S2). In step S2, the stirring unit 120 rotates at 6000 rpm for three minutes.

Even after the main ingredient aqueous solution W1 is completely injected, the stirring unit 120 is further rotated at 6000 rpm for two minutes to homogenize the main ingredient aqueous solution W1 and the polymer oil phase solution O. Through Steps S2 and S3, the main ingredient aqueous solution W1 and the polymer oil phase solution O are homogenized to form a W1/O emulsion.

When the W1/O emulsion is manufactured through the above-described process, a W1/O/W2 emulsion manufacturing process proceeds. Step S4 is carried out in a separate stirring apparatus (not shown) rather than the emulsion homogenization apparatus 100 used in Steps S1 to S3.

In Step S4, while stirring the polymer aqueous solution W2, the W1/O emulsion is injected into the polymer aqueous solution W2 in the form of droplets. Here, the polymer aqueous solution W2 is a solution in which a polymer material, polyvinyl alcohol (PVA), is dissolved in WFI. The polymer aqueous solution W2 is a water phase.

In step S4, while the polymer aqueous solution W2 is being injected and flowing, a stirring unit of a separate stirring apparatus (not shown) rotates at 7200 rpm to stir. The polymer aqueous solution W2 is injected into the stirring container at an injection rate of 1.5 L/min. In addition, the W1/O emulsion is injected at an injection rate of 14.6 mL/min for 80 minutes. At this time, while the polymer aqueous solution W2 is flowing into the stirring container of the stirring apparatus (not shown), the W1/O emulsion is injected into the polymer aqueous solution W2 in the form of droplets.

In Step S5, a W1/O/W2 emulsion is formed by a second homogenization process of the polymer aqueous solution W2 and the W1/O emulsion. The stirring speed in Step S5 is 7200 rpm, which is the same as the stirring speed of Step S4.

After the W1/O/W2 emulsion is prepared through Steps S4 and S5, the microsphere manufacturing step is carried out.

In Step S6, the W1/O/W2 emulsion forms microspheres as the organic solvent volatilizes through an underwater drying process. The W1/O/W2 emulsion is centrifuged, the supernatant of the W1/O/W2 emulsion is discarded, and the microspheres are washed with distilled water and then freeze-dried.

Step S6 is a known technology for manufacturing microspheres, and a detailed description of the underwater drying process, washing process, freeze-drying process, and microsphere pulverizing process will be omitted.

The method for manufacturing sustained-release microspheres having an improved drug encapsulation rate according to one embodiment of the present invention enables mass production of sustained-release microspheres exhibiting a stable effect by increasing the drug encapsulation rate by approximately twice that of the conventional method through homogenization of W1/O emulsion and W1/O/W2 emulsion even when the microspheres are mass-produced.

The preferred embodiments of the present invention described above are disclosed for the purpose of illustration, and those skilled in the art having common knowledge of the present invention will be able to make various modifications, changes, and additions within the spirit and scope of the present invention, and such modifications, changes, and additions should be considered to fall within the scope of the following claims.

### [Industrial Applicability]

According to the emulsion homogenization apparatus according to at least one embodiment of the present invention, while stirring a polymer oil phase solution O in advance, a main ingredient aqueous solution can be injected in the form of droplets under stirring blades in a stirring container, and as a result, the main ingredient aqueous solution W1 and the polymer oil phase solution O are homogeneously stirred, thereby homogenizing a W1/O emulsion.

## Claims

1. An emulsion homogenization apparatus comprising:
a stirring container;
a stirring unit including a stirring shaft and stirring blades mounted on the stirring shaft, the stirring blades being rotatably provided within the stirring container; and
a medicinal fluid injection part having a discharge port through which a main ingredient aqueous solution including a drug is discharged in the form of droplets,
wherein the discharge port is located between a bottom surface of the stirring container and the stirring blades and disposed to face the stirring blades.

2. The apparatus of claim 1, wherein the medicinal fluid injection part is provided to discharge the main ingredient aqueous solution in the form of droplets toward the stirring blades.

3. The apparatus of claim 2, wherein the medicinal fluid injection part has a discharge port spaced apart from the stirring blades.

4. The apparatus of claim 1, further comprising a control unit controlling the stirring unit and a medicinal fluid supply part,
wherein the control unit is provided to perform stirring of a polymer oil phase solution by rotating the stirring blades at a first speed when the polymer oil phase solution is accommodated in the stirring container before the main ingredient aqueous solution is supplied through the medicinal fluid injection part.

5. The apparatus of claim 4, wherein the control unit supplies the main ingredient aqueous solution into the stirring container through the medicinal fluid supply unit and increases the rotation speed of the stirring blades to a second speed greater than the first speed while stirring the polymer oil phase solution.

6. The apparatus of claim 1, wherein the medicinal fluid injection part includes an injection tube.

7. The apparatus of claim 5, wherein the injection tube is bent at least twice within the stirring container.

8. The apparatus of claim 1, wherein the bottom surface of the stirring container is provided with a body through-hole at a position facing the stirring blades, and the medicinal fluid injection part is inserted into the stirring container through the body through-hole, and the discharge port is installed facing the stirring blades.

9. The apparatus of claim 1, wherein the stirring unit has a stirring through-hole passing through the stirring shaft and the stirring blades, the medicinal fluid injection part is disposed within the stirring unit along the stirring through-hole, and the discharge port is provided to pass through the stirring through-hole and be exposed under the stirring blades.

10. A method of manufacturing sustained-release microspheres having an improved drug encapsulation rate, the method comprising:
S1) a step of stirring a polymer oil phase solution (O), which is an oil phase, using the emulsion homogenization apparatus of claim 1;
S2) a step of injecting a main ingredient aqueous solution (W1) in the form of droplets into the polymer oil phase solution (O) being stirred;
S3) a step of forming a W1/O emulsion by a first homogenization process of the main ingredient aqueous solution (W1) and the polymer oil phase solution (O);
S4) a step of injecting the W1/O emulsion in the form of droplets into a polymer aqueous solution (W2) while stirring the polymer aqueous solution (W2);
S5) a step of forming a W1/O/W2 emulsion by a second homogenization process of the polymer aqueous solution (W2) and the W1/O emulsion; and
S6) a step of forming microspheres by an underwater drying process of the W1/O/W2 emulsion.

11. The method of claim 10, wherein in Step S2, the droplets of the main ingredient aqueous solution (W1) are injected under the stirring unit of the emulsion homogenization apparatus and mixed with the polymer oil phase solution (O) while being dispersed by the vortex of the polymer oil phase solution (O).

12. The method of claim 10, wherein in Step S2, the polymer oil phase solution (O) is stirred at a speed 1.5 times faster than that of Step S1.

13. The method of claim 10, wherein in Step S4, the W1/O emulsion is injected for a preset time at a rate within a range that is 1/100 slower than the injection rate of the polymer aqueous solution (W2) while the polymer aqueous solution (W2) is flowing into the stirring container.

14. The method of claim 10, wherein the polymer oil phase solution (O) is a solution in which a polymer material, polylactic acid (PLA) or poly lactic-co-glycolic acid (PLGA), is dissolved in methylene chloride (MC).

15. The method of claim 10, wherein the polymer aqueous solution (W2) is a solution in which a polymer substance, polyvinyl alcohol (PVA), is dissolved in water for injection (WFI).

16. The method of claim 10, wherein the main ingredient aqueous solution (W1) is a solution in which a drug is dissolved in WFI.

17. The method of claim 16, wherein the drug is leuprolide acetate, and the chemical formula of the leuprolide acetate is (2S)-N-[(2S)-1-[[(2S)-1-[[(2S)-1-[[(2S)-1-[[(2R)-1-[[(2S)-1-[[(2S)-5-(diaminomethylideneamino)-1-[(2S)-2-(ethylcarbamoyl)pyrrolidin-1-yl]-1-oxopentan-2-yl]amino]-4-methyl-1-oxopentan-2-yl]amino]-4-methyl-1-oxopentan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide;[(8R,9S,10R,13S,14S,17R)-17-ethynyl-13-methyl-3-oxo-1,2,6,7,8,9,10,11,12,14,15,16-dodecahydrocyclopenta[a]phenanthren-17-yl] acetate.
